# EUROPÄISCHE PATENTSCHRIFT

(11) **EP 0 743 075 B1**
(45) Veröffentlichungstag und Bekanntmachung des Hinweises auf die Patenterteilung: **17.10.2001**
(21) Anmeldenummer: 96107722.9
(22) Anmeldetag: 15.05.1996
(51) Int. Cl.: A61M 16/00

(54) **Beatmungsbeutel**
Resuscitation bag
Ballon d'insufflateur manuel

(30) Priorität: 16.05.1995 DE 19517857
(43) Veröffentlichungstag der Anmeldung: 20.11.1996
(73) Patentinhaber: WILLY RÜSCH AG, D-71394 Kernen (DE)
(72) Erfinder: Lakhani, Mukund, 71717 Beilstein (DE); Brokelmann, Jörg, 74078 Heilbronn-Biberach (DE); Singvogel, Armin, 71686 Remseck (DE)
(74) Vertreter: KOHLER SCHMID + PARTNER

(56) Entgegenhaltungen:
- EP-A- 0 139 363
- EP-A- 0 367 285
- EP-A- 0 598 948
- US-A- 3 363 833
- US-A- 4 374 521
- US-A- 4 501 271

## Beschreibung

Die Erfindung betrifft einen Beatmungsbeutel, bestehend aus einem aufblasbaren, seitlich eingefalteten Reservoir, an dem ein Halteelement und ein ein Öffnungsvolumen bildender Stutzen vorgesehen sind, der mit einer Muffe verbunden ist.

Derartige Beatmungsbeutel aus Gummi werden zur künstlichen Beatmung verwendet.

Wenn ein Kranker oder Verletzter von sich aus nicht mehr in der Lage ist, ausreichend zu atmen, d.h., für eine genügende Sauerstoffaufnahme und Kohlendioxidabgabe zu sorgen, ist eine künstliche Atmung oder Beatmung notwendig. Eine künstliche Beatmung wird auch meist im Zusammenhang mit Narkosen angewandt.

Eine künstliche Beatmung ist sowohl mit als auch ohne Beatmungsgerät durchführbar. Eine Möglichkeit, eine künstliche Beatmung anzuwenden, stellt die Verwendung eines Beatmungsbeutels dar. Da der Beatmungsbeutel seitlich eingefaltet ist, kann der Beatmungsbeutel aufgeblasen und zusammengedrückt werden, um die künstliche Beatmung durchführen zu können. Der bekannte Beatmungsbeutel weist eine Muffe auf, mit der der Beatmungsbeutel an einer Sauerstoffzuleitung angeschlossen und mit einer Atemmaske verbunden werden kann. Der Beatmungsbeutel ist von Hand bedienbar und gehört zur Ausrüstung von Rettungswagen, Operationsräumen u.s.w..

Um den Beatmungsbeutel mit ausreichender Sicherheit und Stabilität, insbesondere in den am stärksten belasteten Bereichen, dem Übergang zwischen Reservoir und Muffe und im Bereich des Halteelementes, herstellen zu können, wird der bekannte Beatmungsbeutel aus Gummi gefertigt. Aufgrund der gummielastischen Eigenschaften genügt der bekannte Beatmungsbeutel auch der Iso-Norm 5362, die für derartige Beatmungsbeutel zwingend vorgeschrieben ist.

Nachteiligerweise kann ein Kontakt mit dem bekannten Beatmungsbeutel aus Gummi aber zu einer Allergie bzw. Überempfindlichkeitsreaktion sowohl beim Patienten als auch bei dem behandelnden Arzt oder Pflegepersonal führen. Wenn der bekannte Beatmungsbeutel aus Latex oder einem ähnlichen Material gefertigt ist, kann es zu sogenannten Latex-Allergien kommen.

Der bekannte Beatmungsbeutel aus Gummi bzw. Latex weist den weiteren Nachteil auf, daß ein derartiges Material altert, und es nach einer gewissen Verwendungsdauer des Beatmungsbeutel zu einer Verminderung seiner elastischen Eigenschaften kommt. Daher neigt der bekannte Beatmungsbeutel nach einer gewissen Einsatzzeit zu Rißbildungen.

Der vorliegenden Erfindung liegt daher die Aufgabe zugrunde, den bekannte Beatmungsbeutel derart weiterzuentwickeln, daß der Beatmungsbeutel aus einem hautverträglichen und keine allergischen Reaktionen hervorrufenden Werkstoff gefertigt ist und die Stabilitäts- und Sicherheitsanforderungen der Iso-Norm 5362 erfüllt.

Die vorliegende Aufgabe wird erfindungsgemäß dadurch gelöst, daß der gesamte Beatmungsbeutel aus Silicon oder einem thermoplastischen Elastomer gefertigt ist, daß das Halteelement einen einvulkanisierten Haltering aufweist, und daß der Stutzen einen umlaufenden Wulst aufweist, der einen Absatz der Muffe hintergreift und mit diesem untrennbar verbunden ist.

Aufgrund der Verwendung eines Materials aus einem Silicon weist der erfindungsgemäße Beatmungsbeutel sowohl bei tiefen als auch bei hohen Temperaturen konstante elastische Eigenschaften auf. Der erfindungsgemäße Beatmungsbeutel ist wärmebeständig und außerdem hydrophob.

Da der erfindungsgemäße Beatmungsbeutel nur aus Teilen besteht, die aus einem Silicon gebildet sind, ist der erfindungsgemäße Beatmungsbeutel physiologisch äußerst verträglich und neigt nicht dazu, Allergien hervorzurufen. Außerdem ist der erfindungsgemäße Beatmungsbeutel aufgrund der Verwendung eines Silicons mehrfach sterilisierbar.

Bisher wurde ein Beatmungsbeutel deshalb nicht aus Silicon gefertigt, da Silicon im Vergleich zu Gummi keine hohe Reißdehnung aufweist. Insbesondere bereitete die Verwendung eines Silicons in den Bereichen des Haltelements und der Muffe des Beatmungsbeutels aufgrund der hohen Materialbelastung in diesen Bereichen größte Probleme. Wenn aber das Haltelement einen einvulkanisierten Haltering aufweist, der beispielsweise bei einem Spritzvorgang, d.h., bei der Fertigung des Beatmungsbeutels, in das Halteelement eingeformt wird, erreicht der erfindungsgemäße Beatmungsbeutel auch in diesem Bereich eine hohe Stabilität, die größten Belastungen Stand hält. Der einvulkanisierte Haltering ist insbesondere aus einem Silikon, einem Thermoplast, einem Elastomer, einem Metall oder einer Keramik herstellbar. Durch den Haltering wird eine Verstärkung des Halteelements erreicht. Eine derartige Verstärkung könnte auch durch einvulkanisierte Elemente, die eine von einem Haltering verschiedene geometrische Form aufweisen, gebildet werden. Da der Stutzen, der das Öffnungsvolumen des Reservoirs des Beatmungsbeutels bildet, einen Absatz der Muffe hintergreift und mit diesem Absatz fest verbunden ist, weist der erfindungsgemäße Beatmungsbeutel auch in diesem zweiten besonders beanspruchten Bereich eine bevorzugte Kombination aus Festigkeit und Elastitizität auf, das ein stabil geöffnetes Lumen gewährleistet. Aus diesen Gründen weist auch der aus Silicon gefertigte Beatmungsbeutel die von der Iso-Norm 5362 geforderten Eigenschaften auf. Eine stabile Verbindung des Stutzen mit der Muffe läßt sich beispielsweise durch eine Klebeverbindung oder ein Verschweißen erreichen.

Durch die Iso-Norm werden auch Anforderungen an die Leckrate des Beatmungsbeutels gestellt. Die Verwendung eines Silicons für einen Beatmungsbeutel bereitete bislang Schwierigkeiten hinsichtlich der Leckrate aufgrund von Rißbildungen in den Übergangsbereichen des Reservoirs zu dem Halteelement und der Muffe. Der erfindungsgemäße Beatmungsbeutel genügt aufgrund der erfindungswesentlichen Ausbildung in diesen Bereichen auch hinsichtlich der Leckrate der Iso-Norm 5362. Dies ist besonders hinsichtlich einer Behandlung eines Patienten mit einem Narkosegas wichtig.

Bei einer besonders bevorzugten Ausführungsform der vorliegenden Erfindung weist der Haltering über den Außenumfang verteilt Noppen auf und ist aus einem Material mit einer höheren Härte nach Shore A oder D als das Reservoir gefertigt. Aufgrund der ausgebildeten Noppen kann der Haltering in eine Spritzform eingelegt werden und kann beim Spritzvorgang vollkommen von dem eingespritzten Material umgeben werden, so daß er formschlüssig mit dem Reservoir verbunden werden kann. Auf diese Art und Weise entsteht ein einteiliger Beatmungsbeutel, der im Bereich des Halteelements eine außerordentlich gute Kombination von Festigkeit und Elastitizität aufweist. Dies wird noch dadurch erhöht, wenn der Haltring aus einem härteren Material als das Reservoir gefertigt ist.

Wenn der Haltering aus einem Material mit einer Härte von 70 Shore A gefertigt ist, weist das Halteelement eine derart hohe Reißfestigkeit auf, daß es bei einem ordnungsgemäßen Gebrauch des erfindungsgemäßen Beatmungsbeutels nicht zu Rißbildungen im Bereich des Halteelementes kommen kann.

Wenn das Reservoir aus einem Material mit einer Härte von 20 bis 70 Shore A gefertigt ist, ist das Reservoir leicht aufblasbar und leicht durch ein Zusammendrücken mit Hilfe der Hände wieder entleerbar.

Weitere Vorteile ergeben sich aus der Beschreibung und der beigefügten Zeichnung. Die erwähnten Ausführungsformen sind nicht als abschließende Aufzählung zu verstehen, sondern haben vielmehr beispielhaften Charakter.

Die Erfindung ist in der Zeichnung dargestellt und wird anhand von Ausführungsbeispielen näher erläutert. Es zeigt:
- Fig. 1: eine Seitenansicht eines erfindungsgemäßen Beatmungsbeutels;
- Fig. 2: eine um 90° gegenüber Fig. 1 gedrehte Seitenansicht des erfindungsgemäßen Beatmungsbeutels;
- Fig. 3: einen Schnitt durch eine Muffe des Beatmungsbeutels nach Fig. 1 und 2,
- Fig. 4: eine Draufsicht eines Halterings, der über den Außenumfang verteilt Noppen aufweist.

Die einzelnen Figuren der Zeichnung zeigen den erfindungsgemäßen Gegenstand teilweise stark schematisiert und sind nicht maßstäblich zu verstehen.

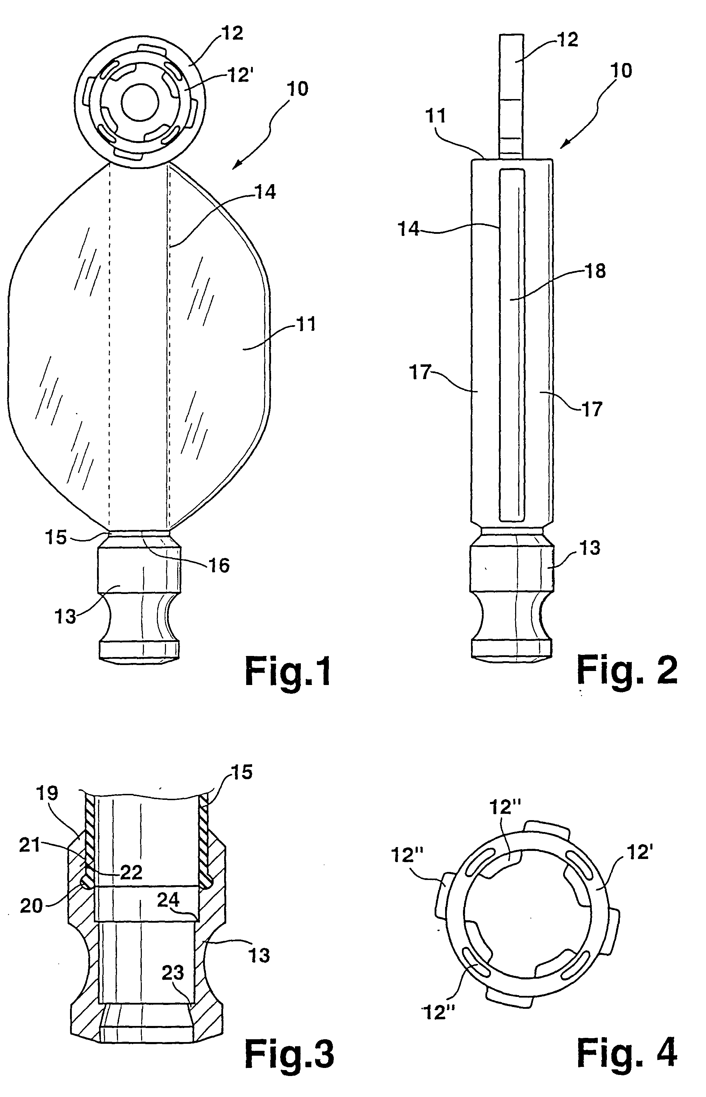

Fig. 1 zeigt eine Seitenansicht des erfindungsgemäßen Beatmungsbeutels 10. Der Beatmungsbeutel 10 weist ein Reservoir 11 auf, das mit einem Haltelement 12 und mit einer Muffe 13 verbunden ist. Das Haltelement 12 ist durchsichtig und ringförmig ausgebildet und besitzt in seinem Inneren einen in der Figur gezeigten Haltering (12') auf, der einvulkanisiert und eingespritzt ist. Aufgrund des Halterings (12') weist das Haltelement (12) eine besonders hohe Reißfestigkeit auf. Das Reservoir 11 besitzt ein aufblasbares und dehnbares Volumen, da es seitlich über zwei Faltlinien 14 eingefaltet ist. Ein Stutzen 15 bildet ein Öffnungsvolumen 16 des Beatmungsbeutels 10. Der Stutzen 15 ist fest mit der Muffe 13 verbunden.

Fig. 2 zeigt eine weitere Seitenansicht des Beatmungsbeutels 10. Das Reservoir 11 wird durch Seitenbereiche 17 gebildet, die voneinander durch einen nach innen gefalteten Faltbereich 18 getrennt sind. Der Faltbereich 18 ist durch Faltlinien 14 nach innen faltbar. Einenends ist an das Reservoir 11 das Halteelement 12 angeformt und andernends ist das Reservoir 11 mit der Muffe 13 fest verbunden.

Fig. 3 zeigt einen vergrößerten Schnitt durch die Muffe 13. Zum Reservoir 11 hin weist die Muffe 13 einen Abschnitt 19 auf, der von einem umlaufenden Wulst 20 des Stutzens 15 hintergriffen ist. Durch das Hintergreifen des Wulstes 20 wird der Stutzen 15 an der Muffe 13 verankert. Dabei liegt die Außenwand 21 des Stutzens 15 an einer Innenwand 22 des Absatzes 19 dicht und fest an. Die Verbindung der Außenwand 21 mit der Innenwand 22 kann durch eine Klebeverbindung, ein Vulkanisieren oder eine andere Art der Verbindung zustandekommen, so daß der Stutzen 15 fest mit der Muffe 13 verbunden ist. Weiterhin weist die Muffe 13 Vorsprünge 23 und 24 auf, um ein in die Muffe 13 eingeführtes Muffengegenstück hintergreifen und festhalten zu können. Der Stutzen 15 kann im Bereich Muffe 13 zusätzlich verstärkt und/oder verdickt sein, um die Reißfestigkeit der Verbindung noch weiter zu erhöhen und einen guten Sitz des Muffengegenstücks zu gewährleisten.

Fig. 4 zeigt einen Haltering 12' in vergrößerter Darstellung. An dem Haltering 12' sind Noppen 12'' ausgebildet, die zum einen radial nach außen vorstehen und radial nach innen weisen. Weiterhin sind die Noppen 12'' am Außenumfang des Halterings 12' auch in axialer Richtung ausgebildet.

Ein Beatmungsbeutel (10) besteht aus einem aufblasbaren, seitlich eingefalteten Reservoir (11). An dem Reservoir sind ein Halteelement (12) und ein ein Öffnungslumen (16) bildender Stutzen (15) vorgesehen, der mit einer Muffe (13) verbunden ist. Der gesamte Beatmungsbeutel (10) ist aus einem Silicon oder thermoplastischen Elastomer hergestellt. Daher ist der Beatmungsbeutel (10) aus einem hautverträglichen und keine allergischen Reaktionen hervorrufenden Werkstoff gefertigt. Das Halteelement (12) weist einen einvulkanisierten Haltering (12') auf. Der Stutzen (15) ist mit einem umlaufenden Wulst (20) versehen, der einen Absatz (19) der Muffe hintergreift und mit diesem untrennbar verbunden ist. Daher erfüllt der Beatmungsbeutel (10) die Stabilitäts- und Sicherheitsanforderungen der Iso-Norm 5362.

## Patentansprüche

1. Beatmungsbeutel (10) bestehend aus einem aufblasbaren, seitlich eingefalteten Reservoir (11), an dem ein Halteelement (12) und ein ein Öffnungslumen (16) bildender Stutzen (15) vorgesehen sind, der mit einer Muffe (13) verbunden ist,
**dadurch gekennzeichnet,**
**daß** der gesamte Beatmungsbeutel (10) aus Silicon oder einem thermoplastischen Elastomer gefertigt ist, daß das Halteelement (12) einen einvulkanisierten Haltering (12') aufweist, und daß der Stutzen (15) mit einem umlaufenden Wulst (20) versehen ist, der einen Absatz (19) der Muffe (13) hintergreift und mit diesem untrennbar verbunden ist.

2. Beatmungsbeutel nach Anspruch 1, **dadurch gekennzeichnet, daß** der Haltering (12') über den Außenumfang verteilt Noppen (12'') aufweist, und daß der Haltering (12') aus einem Material mit einer höheren Härte nach Shore A oder D als das Reservoir (11) gefertigt ist.

3. Beatmungsbeutel nach Anspruch 2, **dadurch gekennzeichnet, daß** der Haltering (12') aus einem Material mit einer Härte von 70 Shore A gefertigt ist.

4. Beatmungsbeutel nach Anspruch 2, **dadurch gekennzeichnet, daß** das Reservoir (11) aus einem Material mit einer Härte von 20 bis 70 Shore A gefertigt ist.

## Claims

1. An artificial respiration bag (10) comprising an inflatable laterally-folded reservoir (11) on which a holding element (12) and a support (15) which forms a lumen opening (16) are formed, the reservoir being connected to a cuff (13), **characterised in that** the entire artificial respiration bag (10) is produced from a silicone or a thermoplastic elastomer, that the holding element (12) has an integrally-vulcanised holding ring (12') and that the support (15) has a surrounding enlargement (20) which engages a shoulder (19) of the cuff (13) and is permanently attached thereto.

2. An artificial respiration bag according to claim 1, **characterised in that** the holding ring (12') has knobs (12'') distributed about its outer circumference and that the holding ring (12') is made from a material with a greater hardness according to Shore A or D than the reservoir (11).

3. An artificial respiration bag according to claim 2, **characterised in that** the holding ring (12') is made from a material with a hardness of 70 Shore A.

4. An artificial respiration bag according to claim 2, **characterised in that** the reservoir (11) is made from a material with a hardness of 20 to 7C Shore A.

## Revendications

1. Poche de respiration artificielle (10) comprenant un réservoir (11) gonflable, replié latéralement, sur lequel sont prévus un élément de retenue (12) et un raccord de conduite (15) qui forme un orifice de passage (16) et qui est assemblé à un manchon (13),
**caractérisée en ce que** la totalité de la poche de respiration artificielle (10) est réalisée en silicone ou en un élastomère thermoplastique, **en ce que** l'élément de retenue (12) comporte une bague de retenue (12') vulcanisée dans celui-ci, et **en ce que** le raccord de conduite (15) est muni d'un bourrelet (20) périphérique, qui s'engage derriére un décrochement (19) du manchon (13) et est assemblé avec celui-ci de manière inamovible.

2. Poche de respiration artificielle selon la revendication 1, **caractérisée en ce que** la bague de retenue (12') comporte des languettes (12") réparties sur la périphéric extérieure et **en ce que** la bague de retenue (12') est réalisée dans un matériau avec une dureté shore A ou D supèrieure à celle du réservoir (11).

3. Poche de respiration artificielle selon la revendication 2, **caractérisée en ce que** la bague de retenue (12') est réalisée dans un matériau avec une dureté égale à 70 shore A.

4. Poche de respiration artificielle selon la revendication 2, **caractérisée en ce que** le réservoir (11) est réalisé dans un matériau avec une dureté allant de 20 à 70 shore A.
